# EUROPEAN PATENT SPECIFICATION

(11) **EP 0 502 100 B1**
(45) Date of publication and mention of the grant of the patent: **28.01.1998**
(21) Application number: 91900484.6
(22) Date of filing: 02.11.1990
(51) Int. Cl.: A61K 39/02

(54) **REDUCED-PROTEIN SUBUNIT VACCINE FOR SWINE DYSENTERY**
IMPFSTOFF GEGEN SCHWEINEDYSENTERIE MIT VERMINDERTEM GEHALT AN PROTEINUNTEREINHEIT
VACCIN A SOUS-UNITES DE PROTEINES REDUITES CONTRE LA DYSENTERIE PORCINE

(30) Priority: 03.11.1989 US 430986
(43) Date of publication of application: 09.09.1992
(73) Proprietor: AMBICO INC., Dallas Center, IA 50063 (US); IOWA STATE UNIVERSITY RESEARCH FOUNDATION, INC., Ames, 1A Iowa 50011-3020 (US)
(72) Inventor: OSTLE, Anthony, G., Dallas Center, IA 50063 (US); WANNEMUEHLER, Michael, J., Ames, IA 50010 (US)
(74) Representative: Andersen, Henrik Rastrup
(86) International application number: PCT/US90/06401
(87) International publication number: WO 91/06316

(56) References cited:
- WO-A-90/02565
- US-A- 4 152 415
- US-A- 4 469 672
- US-A- 4 506 014
- US-A- 4 748 019
- Science, Volume 211, issued 27 March 1981, GOLDMAN et al "Ultrasensitive stain for proteins in polyacrylamide gels shows regional variation in cerebrospinal fluid proteins", see pages 1437-1438.
- Infection and Immunity, Volume 58, No. 12, issued December 1988, WANNEMUEHLER et al., "Characterization of the major outer membrane antigens of Treponema hyodysenteriae", pages 3032-3039, see the entire document.

## Description

### BACKGROUND OF INVENTION

### Field of Invention

The field of this invention relates to the production of a vaccine effective against swine dysentery, a disease of swine caused by an anaerobic spirochete, Treponema hyodysenteriae.

### Information Disclosure Statement

T. hyodysenteriae has been recognized as the primary etiological agent of swine dysentery (1). Although pigs infected by T. hyodysenteriae may recover and be resistant to subsequent infection by T. hyodysenteriae, efforts to induce immunity by parenteral administration of killed cells of T. hyodysenteriae have met with limited success and are generally ineffective in field use (2). Killed whole-cell T. hyodysenteriae bacterins have been developed, but often use mineral-oil base adjuvants (3) or have not involved pre-treatment of the T. hyodysenteriae cells to increase their effectiveness as a bacterin.

The use of live avirulent strains of T. hyodysenteriae as a vaccine have also been unsuccessful in protecting affected pigs (4,5). Combinations of killed T. hyodysenteriae bacterins followed by live avirulent T. hyodysenteriae vaccines have also been attempted (6).

Killed whole-cell bacterins based on T. hyodysenteriae cells produced by conventional methods have been produced and used in the field. However, the efficacy of these preparations is typically low with vaccinates reported having 50% of the incidence of swine dysentery as unvaccinated controls (7). Other preparations of killed T. hyodysenteriae cells administered as bacterins have also had limited success, with death and clinical signs of swine dysentery reported in both the vaccinated and unvaccinated animals (8).

A principal reason for the inadequate efficacy of vaccines studied to date may be the interference of other T. hyodysenteriae antigens with more functionally immunogenic antigens. A strongly immunogenic antigen which is not protective may be so much more immunogenic than a protective antigen that most of the host animal's immune response Is directed against the nonprotective antigen, to the detriment of the host response to the protective antigen. In other words, nonfunctional antigens (those which do not provide disease resistance) may compete with functional antigens. Other reasons for this may be that the functional antigen is concealed by the nonfunctional antigen (sterically) or that antibody produced to the nonprotective antigen blocks the protective antigen which is thus not processed by the host immune system.

Treponema hyodysenteriae is a helical anaerobic spirochaete. As recently as 1988, researchers declared that "little information is available regarding the physiology, cell biochemistry and nutrition" of this organism (13).

Joens, WO90/02565 (p0ubl. 22 March 1990) relates to a method for preparation of a swine dysentery subunit vaccine. This subunit is a protein-enriched carbohydrate mixture of outer-membrane proteins and lipopolysaccharide of Treponema hyodysenteriae, prepared as a salt extract of the outer envelope or the cells. Treatment of the salt extract with either proteinase K or sodium-M-periodate is said to result in loss of immunogenicity in a CF1 mouse model. Protective extracts separated by SDS-PAGE and silver-stained contained bands at 47-43, 35-33, 14-12 and 10-8 kDa.

Lipooligosaccharides (LOS) have been extracted with hot phenol-water from the outer membranes of T. hyodysenteriae serotypes 1 through 7 (14). These LOSs have been used as an antigenic reagent in an enzyme-linked immunosorbent assay for the detection of antibodies to T. hyodysenteriae antigens (16). This left unresolved the question of whether the LOSs were protective antigens suitable for vaccine use. Moreover, some reports suggested that treponemal lipopolysaccharide was toxic.

### SUMMARY OF INVENTION

This invention is based on a method of increasing the effectiveness of killed preparations of T. hyodysenteriae. The method is practiced by the parenteral administration of a vaccine or bacterin containing as the active immunizing agent T. hyodysenteriae cells and subcellular components which have been pretreated to reduce the protein content of the T. hyodysenteriae components. Whole cells, which may be grown in any of several recognized media and under conventional conditions, are lysed and the pelletized lysates are treated to to physically or chemically degrade the protein and thereby to reduce the amount of protein present. This reduced-protein preparation is then administered by parenteral injection to swine, preferably before they are infected with T. hyodysenteriae. The vaccine comprises one or more treponemal flagellar proteins which are substantially resistant to the chemical or physical degradation.

The equivalent of 5 x 10⁹ killed cells of T. hyodysenteriae, treated to reduce the protein content of the bacterin or vaccine, is sufficient to significantly reduce death and morbidity due to swine dysentery caused by T. hyodysenteriae infection. The protection against both death and morbidity due to swine dysentery caused by T. hyodysenteriae infection which is provided by the reduced-protein vaccine or bacterin is superior to that noted with similar preparations of killed T. hyodysenteriae cells or subcellular components which have not been pretreated to reduce the protein content.

The exact nature of the antigen immune response is unknown, although a systemic response to the LOS- or LPS-like component of T. hyodysenteriae cells by both the cellular and humoral immune systems of the swine is likely to be involved. This immune response to the LPS-like component of T. hyodysenteriae cells is likely to be enhanced by the reduction in extraneous proteins from T. hyodysenteriae cells and other sources which may interfere in the elicitation of a specific immune response to the LPS-like component of T. hyodysenteriae (9). However, the immune response obtained with the proteinase-treated, pelletized lysate is superior ot that obtained with purified LPS.

### BRIEF DESCRIPTION OF THE DRAWINGS

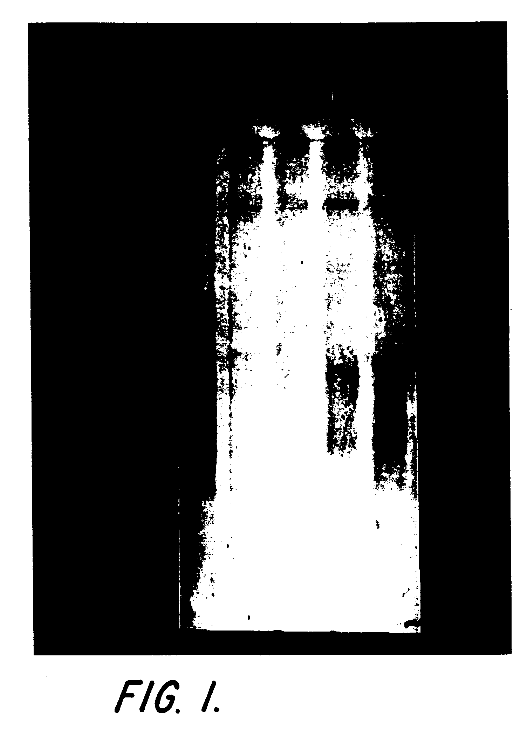

Figure 1 compares the immune response to a PK digest (lanes A, D and E) with that to purified LPS (lanes B and C) in a Western blot assay.

### DETAILED DESCRIPTION OF THE PREFERRED EMBODIMENTS

In one embodiment, the present invention is directed to a vaccine preparation derived from T. hyodysenteriae as defined in the claims, so that a statistically significant reduction in morbidity (bloody scours) is observed in pigs challenged after vaccination. Preferably, the product contains less than 1 µg/ml intact protein of 10,000 daltons or above. Preferably, over 90% of the whole cell lysate proteins larger than 10,000 daltons are reduced to fragments smaller than 10,000 daltons.

Treponema hyodysenteriae strains (both virulent and avirulent) are available from United States and foreign culture depositories and from private culture collections. Several different serotypes (currently seven) of T. hyodysenteriae are known to exist and to cause swine dysentery in susceptible animals. Methods for the isolation of T. hyodysenteriae have been described in the literature (10). Suitable isolates include strain number 31212 (or B204) of serotype 2 from the American Type Culture Collection (a, 11). However, any isolate of T. hyodysenteriae capable of causing disease in swine is suitable for the practice of this invention.

Treponema hyodysenteriae cells may be grown in either solid or liquid growth media. Trypticase soy broth (TSB) with 7.5% horse serum (HS) 0.5% yeast extract, VPI anaerobe salts (12) and 0.05% L-cysteine is suitable for preparing T. hyodysenteriae cells for the practice of this invention, although other suitable media are known in the literature (13). T. hyodysenteriae cells are grown anaerobically or microaerophillically in reduced oxygen atmospheres at temperatures of 35-40° C (13). After growth, T. hyodysenteriae cells may be killed by standard methods such as formalin, merthiolate or heat treatment (although merthiolate is preferred) and collected by centrifugation or ultrafiltration. The T. hyodysenteriae cells may also be killed by the process of lysing the cells which may be performed by physical means such as pressure treatment or sonication or by changes in the pH or tonicity of the surrounding medium.

After suitable T. hyodysenteriae cells are prepared and lysed, the insoluble components of the lysate (e.g., the membrane) are treated to reduce the protein content of the preparation. Such a reduction in the protein content may be accomplished by treatment with protein-degrading enzymes such as proteinase-K. It should be understood that many protein-degrading enzymes are known in the literature and any enzyme or physical or chemical treatment which results in a significant reduction in the protein content of the preparation (without adversely affecting immunogenicity of the product) is suitable for the practice of this method. The treatment may involve use of a combination of enzymes, sequentially or simultaneously, rather than a single enzyme. The specific conditions of temperature, pH and other physical values under which the protein-reduction occurs is dependent on the enzyme or protein-reducing system employed and may be varied, so long as the desired protective effect is obtained. For example, if proteinase-K is used, a commercial proteinase-K preparation is added to a suspension of the pelletized lysate which preferably is then incubated at 56° C for two hours and at 37° C for an additional twelve hours.

The analysis of the proteinase K digested whole cell lysate by SDS-PAGE reveals three to four intact protein bands following coomassie blue staining. These protein bands have an apparent molecular mass of between 30 and 40 kDa (note: these bands would be consistent with the proteins associated with spirochetal flagella). All but one (highest molecular mass) of the proteins can be removed from the preparation by digesting with a 10X concentration of proteinase K. In addition to the above mentioned intact proteins, there are numerous low molecular mass peptides (<10 kDa) observed as a smear in the region of the gel ahead of the dye front. The protein profile of the undigested whole cell lysate consists of many protein bands (>50) with apparent molecular masses between 20 and 150 kDa and no detectable peptide smear near the dye front. Both the PK digest and the whole cell lysate also contain LPS-like material which has three major bands between 16 and 24 kDa. The LPS bands do not stain with Coomassie Blue but are detectable by silver stain. The 18-20 kDa band is the predominant band recognized by antisera following western blot analysis, however, the other two bands are also evident.

The PK digest has been examined by electron microscopy. The electron micrograph revealed intact structures consistent with the treponemal flagella. The composition of the PK digest would also include the bacterial cell wall (peptidoglycan), ribosomes, nucleic acids, and phosphomembranous material. It is believed that the peptidoglycan and other materials may contribute adjuvant-like properties to the vaccine preparation.

Preferably, the reduced-protein suspension of T. hyodysenteriae cells and subcellular components contains the equivalent of at least about 5 x 10⁹ T. hyodysenteriae cells per dose comprises the vaccine, although levels of antigen equal to at least 1 x 10¹⁰ T. hyodysenteriae cells per dose are more preferred. This preparation may be mixed with various adjuvant preparations known in the literature such as mineral oils, metabolizable organic oils, emulsifiers or metal salts to enhance the effectiveness of the vaccine. Preferred adjuvants include Freund's incomplete adjuvant and squalene (Shark-oil). While mineral oils are not preferred, as they are potential carcinogens and may also cause adverse reactions, they may be used if desired. It should be understood that while the use of various adjuvant preparations may enhance the effectiveness of this or any vaccine their use may not be necessary to the practice of this method. The use of adjuvants which favor induction of an IgM response, rather than an IgG response, is preferred.

The vaccine prepared by the practice of this method is administered to swine parenterally, generally by intramuscular or subcutaneous injection. The vaccine is most effective if administered to swine which are otherwise healthy and not already showing symptoms of swine dysentery. The vaccine is preferably administered in a series of at least two doses separated by two-three weeks, but other immunization schedules may be efficacious and may be determined by conventional means.

The method of this invention is further illustrated by the following experimental examples.

### Example 1

A strain of Treponema hyodysenteriae serotype 2 isolated from swine and designated as B-204 was grown in pure culture in a liquid medium consisting of trypticase soy broth supplemented with 5.0% horse serum (HS) 0.5% yeast extract, VPI anaerobe salts (12) and 0.05% L-cysteine. This culture was grown at 39° C in an atmosphere of 10% CO₂, 10% H₂ and 80% N₂ for 12 hours by which time the majority of the growth of T. hyodysenteriae had ceased. The T. hyodysenteriae cells were then killed by the addition of 1:10,000 merthiolate. These killed cells were then collected by centrifugation at 20,000 x g for 45 minutes and resuspended in sterile 0.85% saline after which they were lysed by the application of pressure in a French pressure cell (5,000 psi = 3,52 x 10⁵ g/cm²) and the cell membranes of the lysed T. hyodysenteriae cells collected by centrifugation of the lysate at 100,000 x g for 2 hours. This membrane material was resuspended in sterile phosphate-buffered 0.85% saline (pH 7.2) and treated with protease type XI-S (proteinase-K) at 56° C for 2 hours followed by further incubation at 37° C for 12 hours. After protease treatment the T. hyodysenteriae subcellular components were stored at 4° C and comprised the concentrated vaccine. The concentrated vaccine was mixed with 25% Freund's Incomplete Adjuvant (FIA) to form a water-in-oil-in-water emulsion for injection into test animals. Similar preparations were made with whole-cell untreated T. hyodysenteriae cells in FIA and untreated T. hyodysenteriae cell membranes in FIA to test the effectiveness of the protein-reduction method.

The reduced-protein preparation were tested for outer membrane protein content by sodium-dodecyl-sulfate polyacrylamide gel electrophoresis (SDS-PAGE) assay with untreated T. hyodysenteriae membranes as a control. For a suitable protocol, see Merrill, et al., Science, 211:1437 (1981). The untreated T. hyodysenteriae membranes had many protein bands evident in the gel after silver staining while only three protein bands could be noted in the reduced-protein T. hyodysenteriae membranes. This indicated that the majority of the native or natural T. hyodysenteriae membrane proteins were reduced by the proteinase K treatment to a level below the ability of the SDS-PAGE/silver stain assay to detect specific proteins.

Sixteen SPF (Specific Pathogen Free) swine from a herd known to be free of swine dysentery were weighed and used to test these preparations. Pigs were randomly assigned to groups. Four pigs were given the equivalent of 4 x 10¹⁰ T. hyodysenteriae cells per dose of reduced-protein vaccine in FIA /n two doses two weeks apart. Four pigs were given whole cell T. hyodysenteriae untreated by proteinase K in doses of 2 x 10¹⁰ T. hyodysenteriae cells per dose in FIA in two doses two weeks apart. Four pigs were given membranes untreated by proteinase K in the equivalent of 4 x 10¹⁰ T. hyodysenteriae cells per dose of T. hyodysenteriae vaccine in FIA in two doses two weeks apart. These experimental groups are outlined in Table 1.

**Table 1**

| Experimental Groups of Treatment | | | |
|---|---|---|---|
| Group | Number of pigs | Vaccine | Doses |
| 1 | 4 | 2 X Reduced-Protein Membrane | 2 doses 2 weeks apart |
| 2 | 4 | 1X Whole Untreated Cells | 2 doses 2 weeks apart |
| 3 | 4 | 2 X Untreated Membranes | 2 doses 2 weeks apart |
| 4 | 4 | None (controls) | None |

Ten days after the second vaccination all experimental pigs were fasted for 24 hours, re-weighed and challenged with 1.69 x 10¹¹ actively-growing, motile Treponema hyodysenteriae cells by oral inoculation. Unvaccinated control pigs were in continuous contact with the vaccinated pigs both before and after challenge. All pigs were observed daily for 21 days after challenge and any signs of swine dysentery (eg. bloody diarrhea) were recorded. At the end of the 21 day observation period, all surviving pigs were re-weighed, sacrificed and necropsied. Samples were taken of the intestinal contents of the spiral colon and the cecum for re-isolation of Treponema hyodysenteriae. Lesions typical of swine dysentery were recorded during necropsy.

The results of the post-challenge observations of all groups are outlined in Table 2.

**Table 2**

| Post-Challenge Observations of Vaccinated and Control Pigs | | | | | | | |
|---|---|---|---|---|---|---|---|
| Group | Treatment | #Pigs | MID Total@ | MID Scour@@ | ADG | Mortality | T.hyo* |
| 1 | Reduced-Protein | 4 | 0.0% | 0.0% | 0,44 kg/day (0.98 lb/day) | 0% | 0/4 |
| 2 | Whole-Cell | 4 | 7.2% | 0.0% | 0,21 kg/day (0.47 lb/day) | 0% | 1/4 |
| 3 | Membrane | 4 | 16.1% | 16.1% | 0,39 kg/day (0.87 lb/day) | 0% | 3/4 |
| 4 | CONTROL | 4 | 33.1% | 21.7% | 0,43 kg/day (0.94 lb/day) | 0% | 2/4 |

| | | | | | | | |
|---|---|---|---|---|---|---|---|
| * Treponema hyodysenteriae reisolated from feces. Number of pigs with Treponema hyodysenteriae/number of pigs in group. | | | | | | | |
| @ Total morbidity, all observed symptoms. Equals (total number of days with symptoms/total number of pig days) x 100. | | | | | | | |
| @@ Morbidity with scours only. Difference from total morbidity is chiefly weakness and gauntness counted in the total MID column. | | | | | | | |

### Results

Bloody diarrhea typical of swine dysentery (MID scour) was observed in unvaccinated control animals on 21.7% of the daily observations. Bloody diarrhea was also noted in 16.1% of the daily observations of pigs receiving a vaccine consisting of Treponema hyodysenteriae membranes which had not been treated by the protein reduction method. Pigs receiving whole cell untreated Treponema hyodysenteriae had no bloody diarrhea. None of the four pigs receiving reduced-protein T. hyodysenteriae vaccine had bloody diarrhea typical of swine dysentery. These data clearly demonstrate that the reduced-protein T. hyodysenteriae vaccine was superior in reducing the incidence of bloody scours typical of swine dysentery as compared to unvaccinated control pigs. Further, the protection against bloody diarrhea was significantly greater than that noted in untreated whole-cell Treponema hyodysenteriae vaccines prepared according to reported methods (7).

This protection also extended to increased average daily weight gains in pigs receiving reduced-protein T. hyodysenteriae vaccine (0,44 kg/day) as compared to pigs receiving classical killed, untreated whole-cell T. hyodysenteriae vaccine (0,21 kg/day), although controls were not as severely affected in this test.

Treponema hyodysenteriae was re-isolated after challenge from two of four unvaccinated control pigs (50%) and one of four pigs receiving classical killed, untreated whole-cell T. hyodysenteriae vaccine (25%). However, T. hyodysenteriae was not reisolated from any of the four pigs receiving reduced-protein T. hyodysenteriae vaccine. These data clearly demonstrate that a reduced-protein T. hyodysenteriae vaccine made according to the method in this claim protects pigs vaccinated with such a preparation against the symptoms of swine dysentery (bloody diarrhea), weight loss due to T. hyodysenteriae infection and re-isolation of T. hyodysenteriae. Further, this protection is notably superior not only to unvaccinated control pigs but also to killed whole cell or membrane T. hyodysenteriae vaccines made according to the prior art. The method of reducing the protein content of T. hyodysenteriae vaccines prior to administration to swine clearly results in enhanced protection of swine to swine dysentery due to Treponema hyodysenteriae infection which is superior to that heretofore available.

### Example 2

In an extension of the previous study, thirty SPF (Specific Pathogen Free) swine from a herd known to be free of swine dysentery were weighed and used to test various dosages of preparations derived from reduced-protein membranes, untreated membranes, and whole untreated cells. Pigs were randomly assigned to groups. Six pigs were given the equivalent of 2 x 10¹⁰ T. hyodysenteriae cells per dose of reduced-protein vaccine in FIA in two doses three weeks apart. Six pigs were given whole cell T. hyodysenteriae untreated by proteinase K in doses of 2 x 10¹⁰ T. hyodysenteriae cells per dose in FIA in two doses three weeks apart. Six pigs were given membranes untreated by proteinase K in the equivalent of 2 x 10¹⁰ T. hyodysenteriae cells per dose of T. hyodysenteriae vaccine in FIA in two doses three weeks apart. To test the dose/response of the reduced-protein vaccine, groups of two pigs each were given 4X, 1/2X or 1/4X the dose of reduced-protein vaccine in FIA in two doses three weeks apart. All experimental animals were housed together in one large room and were fed and handled identically. These experimental groups are outlined in Table 3.

**Table 3**

| Experimental Groups and Treatments | | |
|---|---|---|
| Group | Number of Pigs | Vaccine |
| 1 | 2 | 4 X Reduced-Protein Membranes |
| 2 | 6 | 1 X Reduced-Protein Membranes |
| 3 | 2 | 1/2 X Reduced-Protein Membranes |
| 4 | 2 | 1/4 X Reduced-Protein Membranes |
| 5 | 6 | 1 X Whole Untreated Cells |
| 6 | 6 | 1 X Untreated Membranes |
| 7 | 6 | None (controls) |

In each of groups 1-6, two doses of the vaccine were administered 3 weeks apart.

Eight days after the second vaccination all experimental pigs were starved for 24 hours, re-weighed and challenged with 5 x 10¹⁰ actively-growing, motile Treponema hyodysenteriae cells by oral inoculation. This challenge was repeated the next day (day nine after the second vaccination). As all pigs were housed together, the unvaccinated control pigs were in continuous contact with the vaccinated pigs both before and after challenge. All pigs were observed daily for 25 days after the second challenge and any signs of swine dysentery (eg. bloody diarrhea) were recorded. At the end of the 25 day observation period, all surviving pigs were re-weighed, sacrificed and necropsied. Samples were taken of the intestinal contents of the spiral colon, ileo-cecal orifice and the cecum for re-isolation of Treponema hyodysenteriae. Lesions typical of swine dysentery were recorded during necropsy.

The results of the post-challenge observations of all groups are outlined in Table 4.

**Table 4**

| Post-Challenge Observations of Vaccinated and Control Pigs | | | | | | | |
|---|---|---|---|---|---|---|---|
| Vaccine | # Pigs | # Dead | # Bloody Scours | MID* Bloody Scours | MID@ Scours (Other) | T.hyo Reisolated | ADG** kg/day (lb/day) |
| Protein -reduced membrane 4X | 2 | 0 | 0 (0%) | 0% (0%) | 8.0% | 1(50%) | 0,39 (0.86) |
| Protein -reduced membrane 1X | 6 | 0 | 0 (0%) | 0% (0%) | 0.0% | 0(0%) | 0,41 (0.91) |
| Protein -reduced membrane 1/2X | 2 | 0 | 0 (0%) | 0% (0%) | 0.0% | 0(0%) | 0,31 (0.70) |
| Protein -reduced membrane 1/4X | 2 | 1 | 1 (50%) | 5.4% (50%) | 0.0 | 1(50%) | 0,18 (0.40) |
| Whole Cell | 6 (17%) | 1 | 2 (33%) | 7.5% | 8.8% | 3(50%) | 0,29 (0.63) |
| Membrane | 6 (0%) | 0 | 5 (83%) | 16.7% | 4.7% | 5(83%) | 0,18 (0.39) |
| Control | 6 (17%) | 1 | 5 (83%) | 29.6% | 6.3% | 3(50%) | 0,17 (0.37) |

| | | | | | | | |
|---|---|---|---|---|---|---|---|
| * Morbidity Incidence and Duration. Equals (number of days with bloody diarrhea/total number of days since challenge to sacrifice or death) x 100. | | | | | | | |
| @ Morbidity Incidence and Duration. Equals (number of days with diarrhea other than bloody diarrhea/total number of days since challenge to sacrifice or death) x 100. | | | | | | | |
| ** Average Daily Weight Gain. Equals (weight at time of sacrifice or death minus weight at time of challenge)/number of days challenge to sacrifice or death. In pounds gained per day. | | | | | | | |

### Results

Bloody diarrhea typical of swine dysentery was observed in 5/6 (83%) of the unvaccinated control animals. Bloody diarrhea was also noted in 5/6 (83%) pigs receiving a vaccine consisting of Treponema hyodysenteriae membranes which had not been treated by the protein reduction method. Two of six (33%) of pigs receiving whole cell untreated Treponema hyodysenteriae had bloody diarrhea. However, none of the ten pigs receiving reduced-protein T. hyodysenteriae vaccine at either the 4X, 1X or 1/2X doses had bloody diarrhea typical of swine dysentery. When the reduced-protein T. hyodysenteriae vaccine was reduced to 1/4 of a dose, one of the two pigs receiving this vaccine had bloody diarrhea, indicating a dose-response to the reduced-protein T. hyodysenteriae vaccine. These data clearly demonstrate that the reduced-protein T. hyodysenteriae vaccine was superior in reducing the incidence of bloody scours typical of swine dysentery as compared to unvaccinated control pigs. Further, the protection against bloody diarrhea was significantly greater than that noted in untreated whole-cell Treponema hyodysenteriae vaccines prepared according to the previous art (7).

This protection also extended to increased average daily weight gains in pigs receiving reduced-protein T. hyodysenteriae vaccine (0,41 kg/day) as compared to untreated controls (0,17 kg/day) or pigs receiving classical killed, untreated whole-cell T. hyodysenteriae vaccine (0,29 /day). Death loss was reduced 100% in pigs receiving reduced-protein T. hyodysenteriae vaccine as compared to deaths noted in pigs receiving both untreated whole-cell vaccine and no vaccine (controls).

Pigs receiving 1X reduced protein vaccine (group 3) were protected against challenge as measured by average daily weight gain (p=0.065) and reduced morbidity (Bloody Scours) (p=0.007). Pigs given 1/2X reduced protein vaccine (group 4) were protected against challenge as measured by reduced morbidity (Bloody Scours) (p=0.088) but not average daily weight gain (p=0.317). Pigs given 1/4X reduced protein vaccine (group 5) were not significantly protected against challenge as measured by reduced morbidity (Bloody Scours) (p=0.241) or average daily weight gain (p=0.739). All analysis was by the Kruskal-Wallis one-way analysis of variance. Thus, the reduced protein vaccine offers statistically significant protection at concentration of at least about 1/2X. The product contained less than 1 µg/ml intact protein of 5-8,000 Da or above when used.

Treponema hyodysenteriae was re-isolated after challenge from three of six unvaccinated control pigs (50%) and three of six pigs receiving classical killed, untreated whole-cell T. hyodysenteriae vaccine (50%). However, T. hyodysenteriae was only reisolated from one of ten pigs receiving 4X, 1X or 1/2X reduced-protein T. hyodysenteriae vaccine.

These data clearly demonstrate that a reduced-protein T. hyodysenteriae vaccine made according to the method in this claim protects pigs vaccinated with such a preparation against the symptoms of swine dysentery (death loss, bloody diarrhea), weight loss due to Treponema hyodysenteriae infection and re-isolation of Treponema hyodysenteriae. Further, this protection is notably superior not only to unvaccinated control pigs but also to killed whole cell or membrane T. hyodysenteriae vaccines made according to the prior art. The method of reducing the protein content of T. hyodysenteriae vaccines prior to administration to swine clearly results in enhanced protection of swine to swine dysentery due to Treponema hyodysenteriae infection which is superior to that heretofore available.

Tables 5, 6 and 7 further show the advantage of the Proteinease K digest over whole cell lysate and endotoxin preparations. In Table 5, we see a higher cecal weight and increased freedom from gross lesions. Table 6 reveals that the number of T. hyodysenteriae recovered from vaccinated mice following challenge is most reduced by the PK digest. Finally, Table 7 shows a tendency toward a lower number of colony forming units.

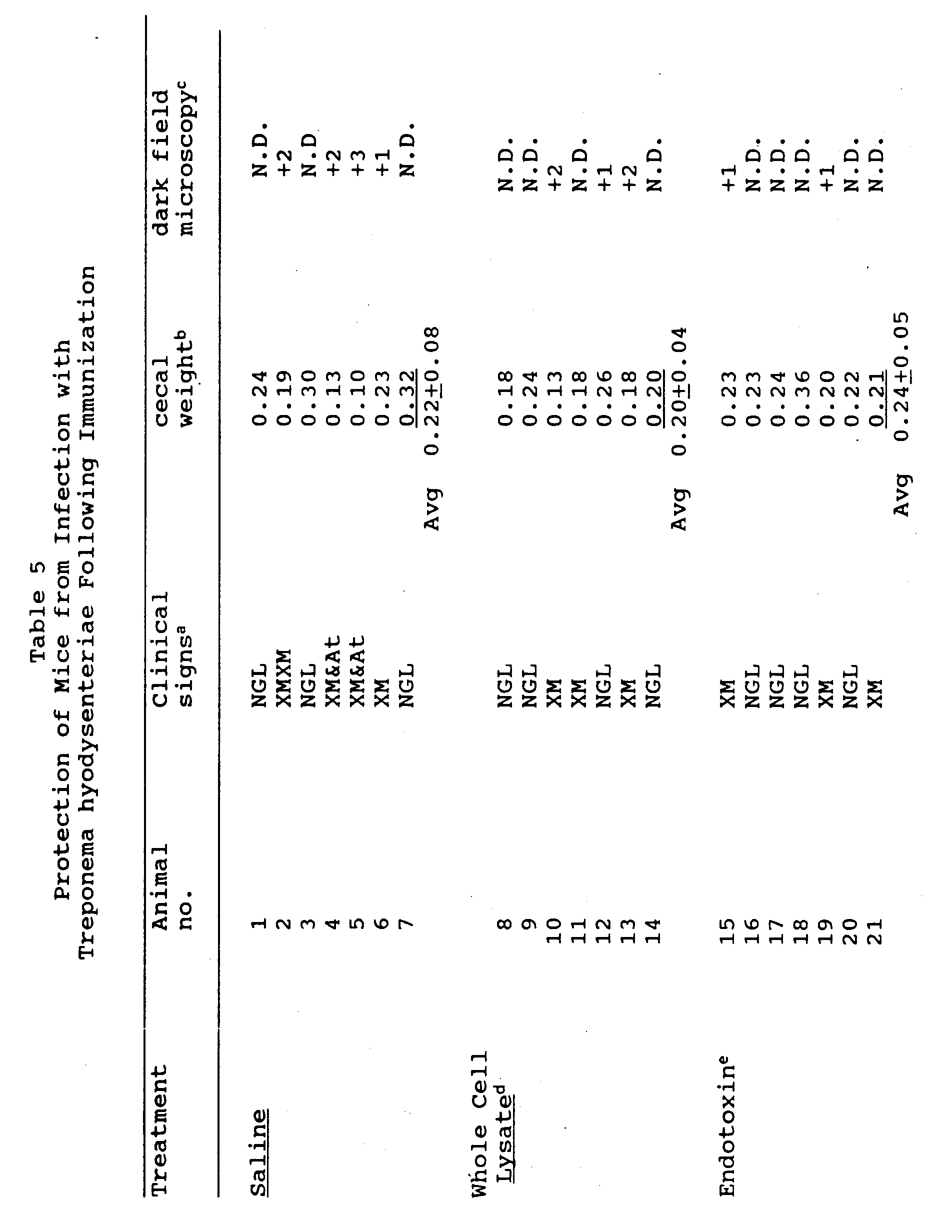

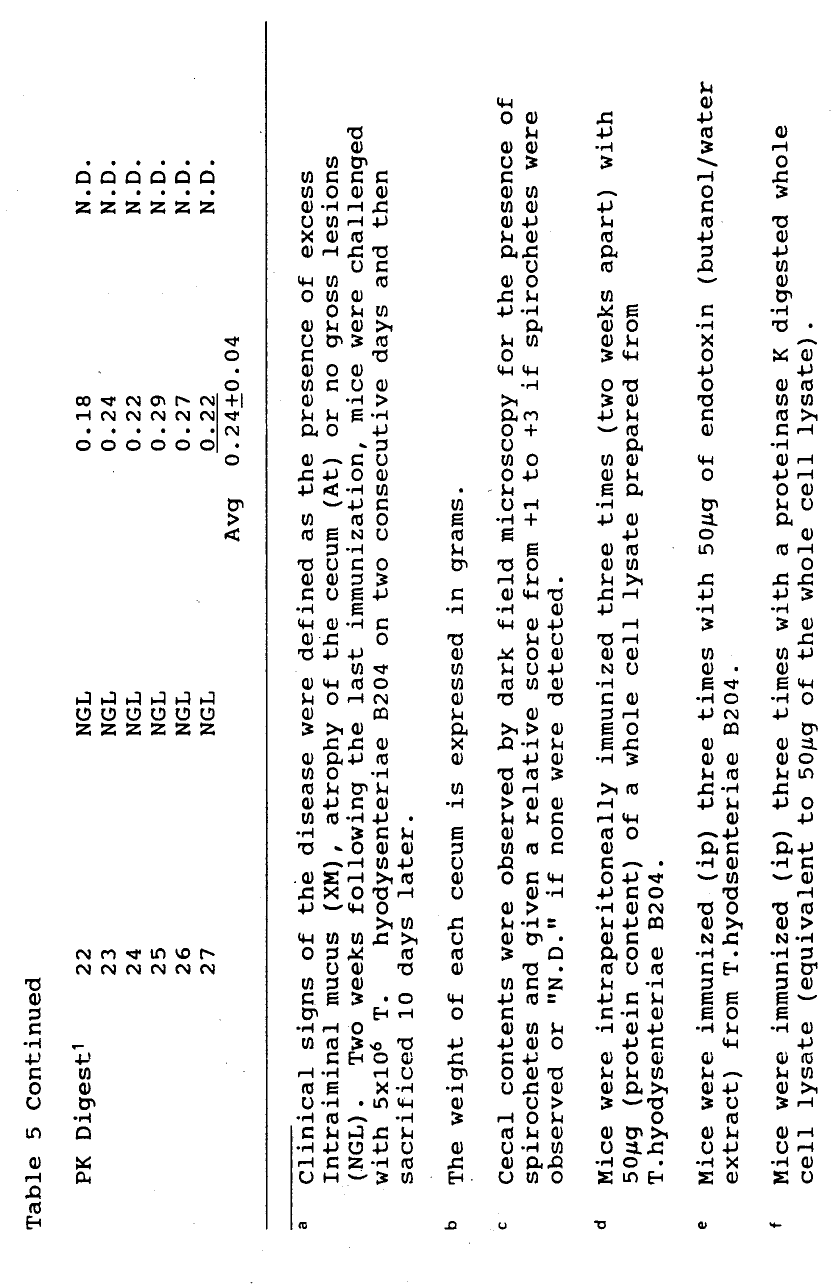

**Table 6**

| Number of Treponema hyodysenteriae recovered from vaccinated mice following Challenge. | | |
|---|---|---|
| Treatment^{b} | n^{c} | CFU^{d} |
| Saline^{a} | 7 | 4.1±2.2 x 10⁶ |
| Whole Cell Lysate | 7 | 2.5±1.1 x 10⁶ |
| Endotoxin | 7 | 1.1±0.5 x 10⁶ |
| Proteinase K Digest | 6 | 5.0±0.5 x 10⁵ |

| | | |
|---|---|---|
| ^{a} Mice challenged on two consecutive days with 5 x 10⁶ T. hyodysenteriae and sacrificed 10 days later. | | |
| ^{b} Mice were immunized as decribed for Table 1. | | |
| ^{c} Number of mice per group. | | |
| ^{d} Colony forming units (CFU) of T. hyodysenteriae per gram of cecal tissue. | | |

**Table 7**

| Colony forming units of T. hyodysenteriae recovered from infected mice. | | | | |
|---|---|---|---|---|
| Treatment | No. | DF^{b} | Culture | CFU^{d} |
| Saline | 1 | - | + | 2.1x10⁴ |
| | 2 | ++ | + | 5.3x10⁶ |
| | 3 | - | + | 1.7x10⁴ |
| | 4 | ++ | + | 6.9x10⁶ |
| | 5 | +++ | + | 1.6x10⁷ |
| | 6 | + | + | 7.0x10⁵ |
| | 7 | - | + | 9.4x10⁴ |
| Whole Cell Lysate^{d} | 8 | - | + | 1.1x10⁵ |
| | 9 | - | + | 8.3x10⁴ |
| | 10 | ++ | + | 2.5x10⁶ |
| | 11 | - | + | 5.3x10⁶ |
| | 12 | + | + | 7.7x10⁶ |
| | 13 | ++ | + | 1.4x10⁶ |
| | 14 | - | + | 1.0x10⁵ |
| Endotoxin | 15 | ⁺ | + | 1.3x10⁶ |
| | 16 | - | - | 0 |
| | 17 | - | + | 8.3x10⁴ |
| | 18 | - | + | <1.0x10⁴ |
| | 19 | + | + | 4.0x10⁶ |
| | 20 | - | + | 1.8x10⁵ |
| | 21 | - | + | 2.0x10⁶ |
| Proteinase K Digest | 22 | - | + | <1.0x10⁴ |
| | 23 | - | + | <1.0x10⁴ |
| | 24 | - | + | 2.3x10⁴ |
| | 25 | - | + | <1.0x10⁴ |
| | 26 | - | + | 3.1x10⁴ |
| | 27 | - | + | 4.5x10⁴ |

| | | | | |
|---|---|---|---|---|
| ^{a} Mice were vaccinated s described for Table 1. | | | | |
| ^{b} Presence (+) or absence (-) of spirochetes in cecal contents as determined by dark field microscopy (DF). | | | | |
| ^{c} Cecal content were directly cultured on blood agar media for the detection of B-hemolytic spirochetes. | | | | |
| ^{d} Ceca were excised from each mouse, weighed, and homogenized. The homogenate was serially diluted and the number of colony forming units (CFU) of T. hyodysenteriae per gram cecum was determined. | | | | |

### Example 3

We have found that serum from pigs vaccinated with our proteinase K-reduced whole cell lysate exhibit immunoreactivity with the 16-20 kDa LPS antigen described in Wannemuehler, et al., Infection and Immunity, 56:3032-39 (Dec. 1988). However, serum from pigs vaccinated with phenol/water extracted LPS did not react with whole cell lysate, suggesting that the purified LPS does not induce an immune response.

Preparation of the Lipopolysaccharide (LPS) of T. Hyodysenteriae T. hyodysenteriae was grown overnight in trypticase soy broth (TSB) supplemented with 5% horse serum, and 1% yeast extract. The cells were harvested by centrifugation, resuspended in phosphate buffered saline and recentrifuged. The cell pellet was then resuspended in 2 volumes (vol/wt) of pyrogen-free water and 1 volume (vol/wt) of 88% phenol. The cell suspension was stirred for 2 hr at room temperature and then for 48 hr. at 4°C. The phenol and water phases were allowed to separate for 7 days at 4°C. The water phase (top) was collected and dialysed against 0.5% NaCl. The solution was then digested with RNAse (10µg/ml) for 2 hr. and then with Proteinase K (10µg/ml) for 2 hr. The solution was then warmed to 68°C and an equal volume of warm 88% phenol was added. The material was stirred at 68°C for 15 min. The solution was then cooled and the phases separated by centrifugation (500 x g). The water was collected and dialysed against 0.5% NaCl. The precipitate was collected by centrifugation (7,500 x g) and washed once with cold ethanol. The precipitate was dissolved in a minimal amount of pyrogen-free water, snap frozen (dry ice and alcohol bath), lyophilized, and stored at -20°C.

Vaccination of Swine with the Proteinase K digested material and LPS Six week old, cross-bred swine (specific pathogen-free) were vaccinated intramuscularly with either 100 µg of treponemal LPS or the Proteinase K digested whole cell lysate (PK digest) at an equivalent dose of 1 x 10¹⁰ cells. Twelve days later the pigs were re-vaccinated with the same preparations. Each antigen dose was incorporated into 0.5 ml of sterile saline and then mixed with 0.5 ml of Freund's incomplete adjuvant (FIA) for a total inoculum of 1.0 ml per pig. Serum was collected 10 days after the second immunization for use in western blot analysis.

Description of Western blot Treponema hyodysenteriae cells were lysed by sonication, snap frozen, and lyophilized. The whole cell lysate (WCL) was resuspended in sterile saline at 1 to 2 mg/ml (wt/vol). The WCL was mixed with Laemmli's treatment buffer, boiled for five minutes, and the material was separated electrophoretically. An amount of WCL containing 250 µg of protein was added to each preparative polyacrylamide gel (12%), Following separation, the antigens were electrophoretically transferred to nylon membranes. The membranes were then blocked with 5% skim milk, washed with Tris buffered saline, and then reacted with the specific antibody samples. Following incubation with the swine antisera, the nylon membranes were washed and reacted with alkaline phosphatase-conjugated goat anti-swine immunoglobulin (µ-specific or c-specific). The substrate solution consisted of Fast Red and Naphthol AS-MX phosphate in Tris buffer (ph 8.0).

The results of the western blot analysis indicated that the swine vaccinated with the PK digest (lanes A, D and E of Fig. 1) developed IgM antibodies to the LPS components of the WCL. However, the LPS vaccinated pigs (lanes B and C) failed to develop LPS-specific antibodies. When the IgG immune response was examined, antibodies were detected to the LPS components as well as to the intact proteins found in the PK digest. Again, the LPS vaccinated pigs did not develop treponemal specific antibodies.

### REFERENCES CITED

a. Glock, et al., U.S. 4,100,272.
b. Goodnow, U.S. 4,152,413.
c. Harris, U.S. 4,152,414.
d. Harris and Goodnow, U.S. 4,152,415.
e. Setsuo, U.S. 4,794,105.
f. Parizek, U.S. 4,758,517.
g. Lysons, U.S. 4,748,019.
h. Glock, U.S. 4,203,968.
i. Gabe, EP 282,965.
j. Coloe, WO88/04555.
k. Parizek, EP 201,976.

1. Harris, D.L., Glock, R. D., Christiansen, C. R. and Kinyon, J. M.. Vet. Med. Small Animal Clin. 67:61 (1972).
2. Joens, L. A., Whipp, S. C., Glock, R. D., and M. E. Neussen. Infect. Immun. 39(1):460 (1983).
3. Fernie , D.S., Ripley, P. H., and Walker, P. D.. Res. Vet. Sci. 35-217 (1983).
4. Hudson, M.J., Alexander, T. J. L., Lysons, R. J. and Wellstead, P. D.. Brit. Vet. J. 130(2):37 (1974).
5. Hudson, M. J., Alexander, T. J. L. , Lysons, R. J. and Prescott, J. F.. Res. Vet. Sci. 21:366 (1976).
6. Lysons, R. J. International Patent No. WO 85/03875. (March, 1985).
7. Parizek, R., Stewart, R., Brown, K. and Blevins, D. Vet. Med. July 1985:80 (1985).
8. Olson, L. D., and Dayalu, K. I.. G. A. Young Swine Conference. 1987:122 (1987).
9. Greer, J. M. and Wannemuehler, M. J.. Infect. Immun. 57(3):717 (1988).
10. Jenkinson, S. R. and Wingar, C. R.. Vet. Rec. 109:384 (1981).
11. Kinyon, J. M., and Harris, D. L.. Vet. Rec. 95:219 (1974).
12. Holdeman, L. V., Cato, E. P., and Moore, W. E. C.. Anaerobe laboratory manual, 4 th Ed. Virginia Polytechnic Institute (1977).
13. Stanton, T. B. and Lebo, D. F. Vet. Microbial. 18:177 (1988).
14. Halter and Joens, Infection and Immunity, 56:3152-56 (Dec. 1988).
15. Wannemuhler, M.J., Hubbard, R.D., and Greer, J.M., Infection and Immunity, 56:3032-39 (Dec. 1988).
16. Joens, L.A., Nord, L.A., Kinyon, J.M., and Egan, I.T., J. Clin. Microbiol. 15:249-52 (1982).

## Claims (Claims for the following Contracting State(s): AT, BE, CH, DE, DK, FR, GB, IT, LI, LU, NL, SE)

1. A vaccine comprising an immunogenic Preparation derived from the insoluble components of the lysate of Treponema hyodysenteriae cells and having a protein content which is substantially reduced relative to the protein content of said insoluble components of the lysate by means of subjecting the insoluble components of the lysate to chemical or physical protein-degradation, the preparation further comprising one or more treponemal flagellar proteins substantially resistant to the chemical or physical degradation, and a pharmaceutically acceptable carrier.

2. The vaccine of claim 1, said preparation comprising a plurality of peptide fragments of one or more treponemal surface proteins as a result of the chemical or physical degradation.

3. The vaccine of any of claims 1 and 2, said preparation further substantially retaining the lipopolysaccharides and lipooligosaccharides associated with the outer membrane of T. hyodysenteriae

4. The vaccine of any of claims 1-3, said preparation further comprising treponemal peptidoglycans, ribosomes, nucleic acids and phosphomembranous material.

5. The vaccine of any of claims 1-4, said preparation being further characterized in that proteins of greater than 10,000 daltons apparent molecular weight, other than proteins having an apparent molecular weight of between 30,000 and 40,000 daltons, are not detectable in such preparation by silver staining under normal loading.

6. The vaccine of any of claims 1-5 wherein the chemical or physical degradation is enzymatic degradation.

7. The vaccine of claim 6, wherein the degradation is by an enzyme having the characteristic substrate specificity of proteinase K.

8. The vaccine of any of claims 1-7, wherein the preparation is derived from an outer membrane extract of such cells.

9. A method of preparing a vaccine according to any of claims 1-8 which comprises enzymatically reducing the protein content of Treponema hyodysenteriae cells or a subcellular membrane component.

10. Use of an antigenic preparation according to any of claims 1-8, in the manufacture of a composition for increasing the resistance of swine to Treponema hyodysenteriae.

## Claims (Claims for the following Contracting State(s): ES, GR)

1. A method of preparing a vaccine comprising an immunogenic preparation derived from the insoluble components of a lysate of Treponema hyodysenteriae cells and having a protein content which is substantially reduced relative to the protein content of said insoluble components of the lysate, the preparation further comprising one or more treponemal flagellar proteins substantially resistant to the chemical or physical degradation, the method comprising subjecting the insoluble components of the lysate to chemical or physical protein-degradation, and mixing the resulting product with a pharmaceutically acceptable carrier.

2. The method of claim 1, wherein said preparation comprises a plurality of peptide fragments of one or more treponemal surface proteins as a result of the chemical or physical degradation.

3. The method of claim 1 or 2, wherein said preparation further substantially retains the lipopolysaccharides and lipooligosaccharides associated with the outer membrane of T. hyodysenteriae.

4. The method of any of claims 1-3, wherein said preparation further comprises treponemal peptidoglycans, ribosomes, nucleic acids and phosphomembranous material.

5. The method of any of claims 1-4, wherein said preparation is further characterized in that proteins of greater than 10,000 daltons apparent molecular weight, other than proteins having an apparent molecular weight of between 30,000 and 40,000 daltons, are not detectable in such preparation by silver staining under normal loading.

6. The method of any of claims 1-5 wherein the chemical or physical degradation is enzymatic degradation.

7. The method of claim 6 which comprises enzymatically reducing the protein content of Treponema hyodysenteriae cells or a subcellular membrane component.

8. The method of claim 6 or 7, wherein the degradation is by an enzyme having the characteristic substrate specificity of proteinase K.

9. The method of any of claims 1-8, wherein the preparation is derived from an outer membrane extract of such cells.

## Patentansprüche (Patentansprüche für folgende(n) Vertragsstaat(en): AT, BE, CH, DE, DK, FR, GB, IT, LI, LU, NL, SE)

1. Impfstoff umfaßend eine immunogene Präparation abgeleitet von den unlöslichen Bestandteilen des Lysats von Treponema hyodysenteriae-Zellen und mit einem Proteininhalt, der im Verhältnis zum Proteininhalt der unlöslichen Bestandteile des Lysats erheblich reduziert ist, dadurch, daß die unlöslichen Bestandteile des Lysats einem chemischen oder physischen Proteinabbau unterworfen werden, welche Präparation außerdem ein oder mehrere treponemale flagellare Proteine, die gegen den chemischen oder physischen Abbau im wesentlichen resistent sind, und einen pharmazeutisch verträglichen Träger umfaßt.

2. Impfstoff nach Anspruch 1, worin die Präparation eine Mehrheit von Peptidfragmenten aus einem oder mehreren treponemalen Oberflächenproteinen infolge des chemischen oder physischen Abbaus umfaßt.

3. Impfstoff nach Anspruch 1 oder 2, worin die Präparation außerdem die Lipopolysaccharide und Lipooligosaccharide, die mit der Außenmembran von T. hyodysenteriae assoziiert sind, im wesentlichen erhält.

4. Impfstoff nach einem der Ansprüche 1-3, worin die Präparation außerdem treponemale Peptidoglykane, Ribosome, Nukleinsäuren und Phosphomembranmaterial umfaßt.

5. Impfstoff nach einem der Ansprüche 1-4, worin die Präparation außerdem dadurch gekennzeichnet ist, daß Proteine mit einem scheinbaren Molekulargewicht von mehr als 10.000 Daltons, mit Ausnahme der Proteine mit einem scheinbaren Molekulargewicht von zwischen 30.000 und 40.000 Daltons, in einer solchen Präparation durch Silberfärbung unter Normalbelastung nicht sichtbar sind.

6. Impfstoff nach einem der Ansprüche 1-5, worin der chemische oder physische Abbau ein enzymatischer Abbau ist.

7. Impfstoff nach Anspruch 6, worin der Abbau mit einem Enzym, das die charakteristische Substratspezifität der Proteinase K hat, stattfindet.

8. Impfstoff nach einem der Ansprüche 1-7, worin die Präparation von einem Außenmembranextrakt solcher Zellen abgeleitet ist.

9. Verfahren zur Herstellung eines Impfstoffes nach einem der Ansprüche 1-8, welches Verfahren die enzymatische Reduktion des Proteininhalts von Treponema hyodysenteriae-Zellen oder einem subzellularen Membranbestandteil umfaßt.

10. Verwendung einer antigenen Präparation nach einem der Ansprüche 1-8 zur Herstellung eines Präparats zur Vergrößerung der Resistenz von Schweinen gegen Treponema hyodysenteriae.

## Patentansprüche (Patentansprüche für folgende(n) Vertragsstaat(en): ES, GR)

1. Verfahren zur Herstellung eines Impfstoffes, umfaßend eine immunogene Präparation abgeleitet von den unlöslichen Bestandteilen des Lysats von Treponema hyodysenteriae-Zellen und mit einem Proteininhalt, der im Verhältnis zum Proteininhalt der unlöslichen Bestandteile des Lysats erheblich reduziert ist, welche Präparation außerdem ein oder mehrere treponemale flagellare Proteine umfaßt, die gegen den chemischen oder physischen Abbau im wesentlichen resistent sind, welches Verfahren umfaßt, daß die unlöslichen Bestandteile des Lysats einem chemischen oder physischen Proteinabbau unterworfen werden, und das resultierende Produkt mit einem pharmazeutisch verträglichen Träger vermischt wird.

2. Verfahren nach Anspruch 1, worin die Präparation eine Mehrheit von Peptidfragmenten aus einem oder mehreren treponemalen Oberflächenproteinen infolge des chemischen oder physischen Abbaus umfaßt.

3. Verfahren nach Anspruch 1 oder 2, worin die Präparation außerdem die Lipopolysaccharide und Lipooligosaccharide, die mit der Außenmembran von T. hyodysenteriae assoziiert sind, im wesentlichen erhält.

4. Verfahren nach einem der Ansprüche 1-3, worin die Präparation außerdem treponemale Peptidoglykane, Ribosome, Nukleinsäuren und Phosphomembranmaterial umfaßt.

5. Verfahren nach einem der Ansprüche 1-4, worin die Präparation außerdem dadurch gekennzeichnet ist, daß Proteine mit einem scheinbaren Molekulargewicht von mehr als 10.000 Daltons, mit Ausnahme der Proteine mit einem scheinbaren Molekulargewicht von zwischen 30.000 und 40.000 Daltons, in einer solchen Präparation durch Silberfärbung unter Normalbelastung nicht sichtbar sind.

6. Verfahren nach einem der Ansprüche 1-5, worin der chemische oder physische Abbau ein enzymatischer Abbau ist.

7. Verfahren nach Anspruch 6, welches die enzymatische Reduktion des Proteininhalts von Treponema hyodysenteriae-Zellen oder einem subzellularen Membranbestandteil umfaßt.

8. Verfahren nach Anspruch 6 oder 7, worin der Abbau mit einem Enzym, das die charakteristische Substratspezifität der Proteinase K hat, stattfindet.

9. Verfahren nach einem der Ansprüche 1-8, worin die Präparation von einem Außenmembranextrakt solcher Zellen abgeleitet ist.

## Revendications (Revendications pour l'(les) Etat(s) contractant(s) suivant(s): AT, BE, CH, DE, DK, FR, GB, IT, LI, LU, NL, SE)

1. Vaccin comprenant une préparation immunogène dérivée des composants insolubles provenant d'un lysat de cellules de Treponema hyodysenteriae et avec un contenu protéique essentiellement reduit par rapport au contenu protéique desdits composants insolubles du lysat, les composants insolubles du lysat ayant subi une dégradation protéique chimique ou physique, ladite préparation comprenant en outre une ou plusieurs protéines tréponémales et flagellaires qui sont essentiellement résistantes à la dégradation chimique ou physique, et un véhicule pharmaceutiquement acceptable.

2. Vaccin selon la revendication 1, ladite préparation comprenant une pluralité de fragments peptidiques provenant d'une ou plusieurs protéines superficielles tréponémales par suite de la dégradation chimique ou physique.

3. Vaccin selon la revendication 1 ou 2, ladite préparation maintenant en outre essentiellement les lipopolysaccharides et les lipooligosaccharides qui sont associés à la membrane extérieure du T. hyodysenteriae.

4. Vaccin selon l'une quelconque des revendications 1 à 3, ladite préparation comprenant en outre des peptidoglycans, des ribosomes, des acides nucléiques et des matériaux phosphomembraneux tréponémaux.

5. Vaccin selon l'une quelconque des revendications 1 à 4, ladite préparation étant en outre caracterisée en ce que des protéines avec un poids moléculaire apparent dépassant 10.000 daltons, autres que des protéines avec un poids moléculaire apparent qui est entre 30.000 et 40.000 daltons, ne sont pas détectables dans une telle préparation par coloration avec de l'argent lors de chargement normal.

6. Vaccin selon l'une quelconque des revendications 1 à 5, dans lequel la dégradation chimique ou physique est une dégradation enzymatique.

7. Vaccin selon la revendication 6, dans lequel la dégradation est avec un enzyme ayant la spécificité caractéristique de la protéinase K pour un substrat.

8. Vaccin selon l'une quelconque des revendications 1 à 7, dans lequel la préparation est dérivée d'un extrait de la membrane extérieure de telles cellules.

9. Procédé de production d'un vaccin selon l'une quelconque des revendications 1 à 8, qui comprend la réduction enzymatique du contenu protéique des cellules de Treponema hyodysenteriae ou un composant membraneux subcellulaire.

10. Utilisation d'une préparation antigénique selon l'une quelconque des revendications 1 à 8 dans la production d'une composition pour l'accroissement de la résistance des cochons au Treponema hyodysenteriae.

## Revendications (Revendications pour l'(les) Etat(s) contractant(s) suivant(s): ES, GR)

1. Procédé de production d'un vaccin comprenant une préparation immunogène dérivée des composants insolubles provenant d'un lysat de cellules de Treponema hyodysenteriae et avec un contenu protéique essentiellement reduit par rapport au contenu protéique desdits composants insolubles du lysat, ladite préparation comprenant en outre une ou plusieurs protéines tréponémales et flagellaires qui sont essentiellement résistantes à la dégradation chimique ou physique, ledit procédé comprenant l'exposition des composants insolubles du lysat à une dégradation protéique chimique ou physique, et le mélange du produit résultant avec un véhicule pharmaceutiquement acceptable.

2. Procédé selon la revendication 1, ladite préparation comprenant une pluralité de fragments peptidiques provenant d'une ou plusieurs protéines superficielles tréponémales par suite de la dégradation chimique ou physique.

3. Procédé selon la revendication 1 ou 2, ladite préparation maintenant en outre essentiellement les lipopolysaccharides et les lipooligosaccharides qui sont associés à la membrane extérieure du T. hyodysenteriae.

4. Procédé selon l'une quelconque des revendications 1 à 3, ladite préparation comprenant en outre des peptidoglycans, des ribosomes, des acides nucléiques et des matériaux phosphomembraneux tréponémaux.

5. Procédé selon l'une quelconque des revendications 1 à 4, ladite préparation étant en outre caracterisée en ce que des protéines avec un poids moléculaire apparent dépassant 10.000 daltons, autres que des protéines avec un poids moléculaire apparent qui est entre 30.000 et 40.000 daltons, ne sont pas détectables dans une telle préparation par coloration avec de l'argent lors de chargement normal.

6. Procédé selon l'une quelconque des revendications 1 à 5, dans lequel la dégradation chimique ou physique est une dégradation enzymatique.

7. Procédé selon la revendication 6, qui comprend la réduction enzymatique du contenu protéique des cellules de Treponema hyodysenteriae ou un composant membraneux subcellulaire.

8. Procéde selon la revendication 6 ou 7, dans lequel la dégradation est avec un enzyme ayant la spécificité caractéristique de la protéinase K pour un substrat.

9. Procédé selon l'une quelconque des revendications 1 à 8, dans lequel la préparation est dérivée d'un extrait de la membrane extérieure de telles cellules.
